(19) Europäisches Patentamt
European Patent Office
Office européen des brevets

(11) Veröffentlichungsnummer: **0 053 678**
**A1**

(12) # EUROPÄISCHE PATENTANMELDUNG

(21) Anmeldenummer: 81108477.1

(22) Anmeldetag: 19.10.81

(51) Int. Cl.³: C 07 D 231/38
A 01 N 43/56
//C07C121/82, A01N37/34

(30) Priorität: 05.12.80 DE 3045904

(43) Veröffentlichungstag der Anmeldung: 16.06.82 Patentblatt 82/24

(84) Benannte Vertragsstaaten: AT BE CH DE FR GB IT LI LU NL SE

(71) Anmelder: **BASF Aktiengesellschaft**
**Carl-Bosch-Strasse 38**
**D-6700 Ludwigshafen(DE)**

(72) Erfinder: **Eicken, Karl, Dr.**
**Waldstrasse 63**
**D-6706 Wachenheim(DE)**

(72) Erfinder: **Plath, Peter, Dr.**
**Berner Weg 24**
**D-6700 Ludwigshafen(DE)**

(72) Erfinder: **Wuerzer, Bruno, Dr.**
**Ruedigerstrasse 13**
**D-6701 Otterstadt(DE)**

(54) **5-Amino-1-phenyl-4-cyanpyrazole, diese enthaltende herbizide Mittel, Verfahren zu ihrer Herstellung und ihre Anwendung als Herbizide.**

(57) 5-Amino-1-phenyl-4-cyanpyrazole der Formel

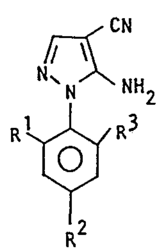

in der $R^1$, $R^2$, $R^3$ Chlor oder Brom bedeuten, mit der Maßgabe, daß $R^1$, $R^2$ und $R^3$ nicht gleichzeitig Chlor bedeuten, und diese enthaltende Herbizide.

EP 0 053 678 A1

Croydon Printing Company Ltd.

5-Amino-1-phenyl-4-cyanpyrazole, diese enthaltende herbizide Mittel, Verfahren zu ihrer Herstellung und ihre Anwendung als Herbizide

Die vorliegende Erfindung betrifft 5-Amino-1-phenyl-4-cyanpyrazole, ein Verfahren zur Herstellung dieser Pyrazole, Herbizide, welche diese Verbindungen enthalten, sowie Verfahren zur Bekämpfung unerwünschten Pflanzenwuchses mit diesen Verbindungen.

5-Amino-1-phenyl-4-cyanpyrazole, die ein bis drei Chloratome im Phenylrest enthalten, z.B. das 5-Amino-4-cyan-1-(2,4,5-trichlorphenyl)-pyrazol sind aus der Literatur bekannt (Eur. J. Med. Chem. (1979) 14, 539-540; J. Heterocycl. Chem. (1975) 12, 1199; J. Heterocycl. Chem. (1980) 17, 265). Die Verbindungen dienen als Zwischenprodukte für die Synthese von Heterocyclen.

Über herbizide Eigenschaften dieser Verbindungen ist nichts bekannt. Lediglich über antibakterielle Wirkung einiger Substanzen ist berichtet worden.

Es wurde nun gefunden, daß 5-Amino-1-phenyl-4-cyanpyrazole der Formel I

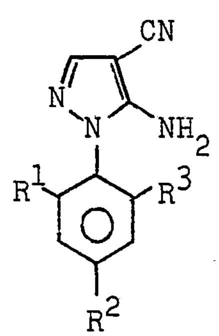

I,

in der $R^1$, $R^2$, $R^3$ für Chlor oder Brom steht, mit der Maßgabe, daß $R^1$, $R^2$ und $R^3$ nicht gleichzeitig Chlor bedeuten,

Sws/P

eine überraschend starke und gleichzeitig selektive herbizide Wirkung aufweisen.

Die neuen Wirkstoffe sind je nach Zielsetzung geeignet zur totalen Bekämpfung des Pflanzenwuchses oder zur selektiven Eliminierung von unerwünschten Pflanzen in bestimmten Kulturpflanzenbeständen.

Die neuen 5-Amino-1-phenyl-4-cyanpyrazole der Formel I können durch Umsetzung substituierter Phenylhydrazine der Formel II

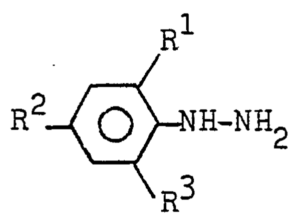

II,

in der $R^1$, $R^2$, $R^3$ für Chlor oder Brom steht, mit Alkoximethylemalodinitrilen der Formel III

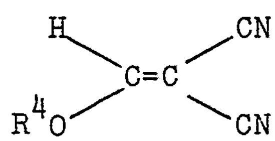

III,

in der $R^4$ für Alkyl mit bis zu 6 Kohlenstoffatomen steht, über die Zwischenstufe IV hergestellt werden. Die 2-Phenylhydrazinomethylenmalodinitrile der Formel IV

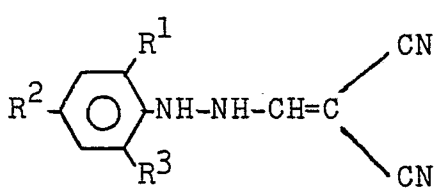

IV,

in der $R^1$, $R^2$, $R^3$ für Chlor oder Brom steht, können gegebenenfalls isoliert werden und besitzen ebenfalls herbizide Eigenschaften.

Zur Herstellung der neuen 5-Amino-1-phenyl-4-cyanpyrazole der Formel I ist es zweckmäßig, die Reaktion in einem Lösungsmittel durchzuführen. Hierzu eignen sich Kohlenwasserstoffe wie Toluol, Xylol; Ether wie Tetrahydrofuran, Dioxan, Glykoldimethylether, Anisol; und insbesondere Alkohole wie Methanol, Ethanol, Propanole, Butanole. Auch Gemische dieser Lösungsmittel können verwendet werden.

Die Reaktion kann bei Temperaturen oberhalb von Raumtemperatur (20°C) durchgeführt werden. Zur Beschleunigung der Umsetzung ist es vorteilhaft beim Siedepunkt des Lösungsmittels bzw. des Lösungsmittelgemisches, vorzugsweise im Bereich zwischen 50 bis 150°C zu arbeiten. Nach Ende der Reaktion wird die Mischung gekühlt und das neue 5-Amino-1-phenyl-4-cyanpyrazol der Formel I isoliert.

Es ist bereits sehr rein, kann jedoch durch erneutes Umkristallisieren weiter gereinigt werden.

Das Alkoximethylenmalodinitril der Formel III wird in mindestens molarer Menge, bezogen auf das Phenylhydrazin der Formel II, eingesetzt.

Das folgende Beispiel erläutert die Herstellung der neuen 5-Amino-1-phenyl-4-cyanpyrazole der Formel I. In dem Beispiel verhalten sich Gewichtsteile zu Volumenteilen wie Kilogramm zu Liter.

Beispiel 1

475 Gewichtsteile 2,4,6-Tribromphenylhydrazin und 275 Gewichtsteile Ethoximethylenmalodinitril werden in 2600 Volumenteilen Ethanol unter Rühren eingetragen. Nach Abklingen einer schwach exothermen Reaktion (Temperaturanstieg auf ca. 30°C) wird 4 Stunden am Rückfluß erhitzt und danach auf 0°C gekühlt.

Durch Absaugen erhält man nach Trocknen im Vakuum bei 40°C 5-Amino-1-(2,4,6-tribromphenyl)-4-cyanpyrazol vom Fp. 196°C (Wirkstoff Nr. 1).

Entsprechend können beispielsweise folgende 5-Amino-1-phenyl-4-cyanpyrazole hergestellt werden:

| Wirkstoff Nr. | | Fp (°C) |
|---|---|---|
| 2 | 5-Amino-1-(2,4-dichlor-6-bromphenyl)-4-cyanpyrazol | 218 |
| 3 | 5-Amino-1-(2-chlor-4,6-dibromphenyl)-4-cyanpyrazol | 184 |
| 4 | 5-Amino-1-(2,6-dichlor-4-bromphenyl)-4-cyanpyrazol | 196 |
| 5 | 5-Amino-1-(4-chlor-2,6-dibromphenyl)-4-cyanpyrazol | 188 |

Vorschrift für die Isolierung von 2-Phenylhydrazinmonomethylenmalodinitril der Formel IV:

61,0 Gewichtsteile Ethoximethylenmalodinittril und 105,8 Gewichtsteile 2,4,6-Tribromphenylhydrazin werden in 500 Volumenteilen Ethanol 16 Stunden bei 25-28°C gerührt. Nach dem Absaugen und Trocknen der Kristalle im Vakuum bei Raumtemperatur erhält man 2-(2,4,6-Tribromphenyl)-hydrazino-methylenmalodinitril.

Die Anwendung als Herbizide erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Herbizide enthalten z.B. 5 bis 95 % (Gew.%) insbesondere 10 bis 80 % Wirkstoff.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes Isooctylphenol-, Octylphenol-, Nonylphenol, Alkylphenolpolyglykoläther. Tributylphenylpolyglykolether, Alkylarylpolyätheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produk-

te, wie Getreidemehle, Baumrinden-, Holz- und Nußschalenmehl, Cellulosepulver und andere feste Trägerstoffe.

Beispiel a

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel b

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-mono-äthanolamid, 2 Gewichtsteilen Calciumsalz der Dodecylbenzolsulfonsäure und 2 Gewichtsteilen des Anlagerungsproduktes von 40 Mol Äthylenoxid an 1 Mol Ricinusöl besteht.

Beispiel c

20 Gewichtsteile der Verbindung 3 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Äthylenoxid an 1 Mol Isooctylphenol und 5 Gewichteilen des Anlagerungsproduktes von 40 Mol Äthylenoxid an 1 Mol Ricinusöl besteht.

Beispiel d

20 Gewichtsteile der Verbindung 4 werden in einer Mischung gelöst, die aus 25 Gewichtsteilen Cyclohexanon, 65 Gewichtsteilen einer Mineralölfraktion vom Siedepunkt 210

bis 280°C und 10 Gewichtsteilen des Anlagerungsproduktes von 40 Mol Äthylenoxid an 1 Mol Ricinusöl besteht.

Beispiel e

80 Gewichtsteile des Wirkstoffs 5 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel f

5 Gewichtsteile der Verbindung 1 werden mit 95 Gewichtsteilen feinteiligem Kaolin innig vermischt. Man erhält auf diese Weise ein Stäubemittel, das 5 Gewichtsprozent des Wirkstoffs enthält.

Beispiel g

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser

erhält man eine wäßrige Dispersion, die 0,04 Gewichtsprozent Wirkstoff enthält.

Beispiel i

20 Teile des Wirkstoffs 1 werden mit 12 Teilen Calciumsalz der Dodecylbenzolsulfonsäure, 8 Teile Fettalkohol-polyglykoläther, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Der Einfluß von Vertretern der neuen 5-Amino-1-phenyl-4-cyanpyrazole auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmiger Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie

danach. Zur Nachauflaufbehandlung wurden entweder direkt gesät und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmengen für die Nachauflaufbehandlung betrugen 0,125; 0,25 und 0,5 kg/ha Wirkstoff für die neue Verbindung Nr. 1. Für die bekannten Verbindungen 5-Amino-1-(2,4,5-trichlorphenyl)-4-cyanpyrazol (A) und 5-Amino-1-(2,3-dichlorphenyl)-4-cyanpyrazol (B) wurden 0,5 kg/ha Wirkstoff eingesetzt. Eine Abdeckung unterblieb bei der Nachauflaufbehandlung. Die Aufstellung der Versuche erfolgte im Gewächshaus, wobei für wärmeliebende Arten wärmere Bereiche (20 bis 40°C) und für solche gemäßigter Klimate 15 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Diese Gewächshausversuche ergeben, daß die Verbindungen Nr. 1, 2, 3, 4 und 5 bei Vorauflaufanwendung von 3,0 kg Wirkstoff/ha eine beachtliche herbizide Wirkung haben.

In diesen Gewächshausversuchen haben die Verbindungen A und B bei Nachauflaufanwendung mit 0,5 kg Wirkstoff/ha ebenfalls eine selektive herbizide Wirkung, welche jedoch im Vergleich zu Verbindung Nr. 2 wesentlich schwächer ausfällt.

Die Gewächshausversuche ergeben ferner, daß die Verbindung Nr. 1 bei Nachauflaufanwendung von 0,5 kg Wirkstoff/ha

breitblättrige unerwünschte Pflanzen bekämpft und gleichzeitig Weizen nicht schädigt.

Ferner zeigen diese Gewächshausversuche, daß die Verbindung Nr. 3 bei Nachauflaufanwendung von 0,5 kg Wirkstoff/ha eine herbizide Wirkung hat, ohne die Kulturpflanzen Reis und Weizen zu schädigen.

Desgleichen bekämpft Verbindung Nr. 5 in den Gewächshausversuchen bei Nachauflaufanwendung von 0,125 kg Wirkstoff/ha einige spezielle breitblättrige Unkräuter und bleibt dabei selektiv für Weizen.

Sind gewisse Kulturpflanzen bei Blattbehandlung gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auch die Blätter darunter wachsender unerwünschter Pflanzen oder die unbedeckte Bodenfläche gelangen (post directed, lay-by). In Anbetracht der Vielseitigkeit der Applikationsmethode können die erfindungsgemäßen Herbizide noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken:

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Aventa sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. naprobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor-Färbedistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea cane phora, Coffea liberica) | Kaffee | coffee plants |
| Cucumis melo | Melone | melons |
| Cucumis sativus | Gurke | cucumber |

| Botanischer Name | Deutscher Name | Englischer Name |
|---|---|---|
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersiocon lycopersicum | Tomate | tomato |
| Malus spp. | Apfel | apple trees |
| Manihot esculenta | Maniok | cassava |
| Medicago sativa | Luzerne | alfalfa (lucerne) |

| Botanischer Name | Deutscher Name | Englischer Name |
|---|---|---|
| Mentha piperita | Pfefferminze | peppermint |
| Musa spp. | Obst- u. Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beens, dry beans |
| Pennisetum glaucum | Perl- oder Rohrkolbenhirse | |
| Petroselinim crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Prunus communis | Birne | pear trees |

| Botanischer Name | Deutscher Name | Englischer Name |
| --- | --- | --- |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potato |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium carymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung synergistischer Effekte können die neuen 5-Amino-1-phenyl-4-cyanpyrazole mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3, 1-Benzoxazinderivate, Benzothiadiazinone, 2,6-Dinitroaniline, N-Phenylcarbamate, Thiolcarbamate, Halogencarbonsäuren Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-m.trifluormethylphenyl-3(2H)-pyridazinon
5-Methylamino-4-chlor-2-(3.α,α,ß,ß-tetrafluoräthoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3.trifluormethylphenyl)-3(2H)-pyridazinon
5-Methoxy-4-chlor-2-(3.trifluormethylphenyl)-3(2H)-pyridazinon
5-Amino-4-brom-2-(3.methylphenyl)-3(2H)-pyridazinon

3-(1-Methylethyl)-1H-2,1,3-benzothiadiazin-4(3H)-on-2,2-dioxid und Salze

3-(1-Methylethyl)-8-chlor-1H-2,1,3-benzothiadiazin-4(3H)--on-2,2-dioxid und Salze

3-(1-Methylethyl)-8-fluor-1H-2,1,3-benzothiadiazin-4(3H)--on-2,2-dioxid und Salze

3-(1-Methylethyl)-8-methyl-1H-2,1,3-benzothiadiazin-4(3H)--on-2,2-dioxid und Salze

1-Methoxymethyl-3-(1-methylethyl)-2,1,3-benzothiadiazin--4(3H)-on-2,2-dioxid

1-Methoxymethyl-8-chlor-3-(1-methylethyl)-2,1,3-benzothiadiazin-4(3H)-on-2,2-dioxid

1-Methoxymethyl-8-fluor-3-(1-methylethyl)-2,1,3-benzothiadiazin-4(3H)-on-2,2-dioxid

1-Cyan-8-chlor-3-(1-methylethyl)-2,1,3-benzothiadiazin--4(3H)-on-2,2-dioxid

1-Cyan-8-fluor-3-(1-methylethyl)-2,1,3-benzothiadiazin--4(3H)-on-2,2-dioxid

1-Cyan-8-methyl-3-(1-methylethyl)-2,1,3-benzothiadiazin--4(3H)-on-2,2-dioxid

1-Cyan-3-(1-methylethyl)-2,1,3-benzothiadiazin-4(3H)-on--2,2-dioxid

1-Azidomethyl-3-(1-methylethyl)-2,1,3-benzothiadiazin--4(3H)-on-2,2-dioxid

3-(1-Methylethyl)-1H-(pyridino-[3,2-e]2,1,3-thiadiazin--(4)-on-2,2-dioxid

N-(1-Ethylpropyl)-2,6-dinitro-3,4-dimethylanilin

N-(1-Methylethyl)-N-ethyl-2,6-dinitro-4-trifluormethyl--anilin

N-n-Propyl-N-ß-chlorethyl-2,6-dinitro-4-trifluormethyl--anilin

N-n-Propyl-N-cyclopropylmethyl-2,6-dinitro-4-trifluor--methyl-anilin

N-Bis-(n-propyl)-2,6-dinitro-3-amino-4-trifluormethylanilin

N-Bis-(n-propyl)-2,6-dinitro-4-methyl-anilin

N-Bis-(n-propyl)-2,6-dinitro-4-methylsulfonyl-anilin
N-Bis-(n-propyl)-2,6-dinitro-4-aminosulfonyl-anilin
Bis-(ß-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert-butyl-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat

Ethyl-N-(3-N'-3-chlor-4-fluorphenylcarbamoyloxy)-phenyl)-carbamat

Ethyl-N-(3-N'-3,4-difluorphenylcarbamoyloxy)-phenyl)-
-carbamat
Methyl-N-(3-(N'-3,4-difluorphenylcarbamoyloxy)-phenyl)-
-carbamat

N-3-(4-Fluorphenoxycarbonylamino)-phenyl-carbaminsäure-
-methylester
N-3-(2-Methylphenoxycarbonylamino)-phenyl-carbaminsäure-
-ethylester
N-3-(4-Fluorphenoxycarbonylamino)-phenyl-thiolcarbaminsäure-
-methylester
N-3-(2,4,5-Trimethylphenoxycarbonylamino)-phenyl-thiolcarbaminsäure-methylester
N-3-(Phenoxycarbonylamino)-phenyl-thiolcarbaminsäure-methylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallyl-
ester
N,N-Di-isopropyl-thiolcarbaminsäure-3-methyl-5-isoxazolyl-
-methylester
N,N-Di-isopropyl-thiolcarbaminsäure-3-ethyl-5-isoxazolyl-
-methylester
N,N-Di-sec.-butyl-thiolcarbaminsäure-ethylester
N,N-Di-sec.-butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethyl-
ester
S-(2,3-Dichlorallyl)-(2,2,4-trimethyl-azetidin)-1-carbo-
thiolat
S-(2,3,3-Trichlorallyl)-(2,2,4-trimethyl-azetidin)-1-
-carbothiolat

S-Ethyl-hexahydro-1-H-azepin-1-carbothiolat
S-Benzyl-(3-methyl-hexahydro-1-H-azepin-1)-carbothiolat
S-Benzyl-(2,3-dimethylhexahydro-1-H-azepin-1)-carbothiolat
S-Ethyl-(3-methylhexahydro-1-H-azepin-1)-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,ß,ß-Tetrafluorpropionsäure-Natriumsalz
α-Methyl-α,ß-dichlorpropionsäure-Natriumsalz
α-Chlor-ß-(4-chlorphenyl)-propionsäure-methylester
α,ß-Dichlor-ß-phenylpropionsäure-methylester
Benzamido-oxy-essigsäure

| | |
|---|---|
| 2,3,5-Trijodbenzoesäure | (Salze, Ester, Amide) |
| 2,3,6-Trichlorbenzoesäure | (Salze, Ester, Amide) |
| 2,3,5,6-Tetrachlorbenzoesäure | (Salze, Ester, Amide) |
| 2-Methoxy-3,6-dichlorbenzoesäure | (Salze, Ester, Amide) |
| 2-Methoxy-3,5,6-trichlorbenzoe-säure | (Salze, Ester, Amide) |
| 3-Amino-2,5,6-trichlorbenzoe-säure | (Salze, Ester, Amide) |

O,S-Dimethyl-tetrachlor-thioterephthalat
Dimethyl-2,3,5,6-tetrachlor-terephthalat
Di-natrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester

2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester
2-Chlor-4-ethylamino-6-isopropylamino-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert-butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.-butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.-Butyl-5-chlor-6-methyluracil
3-tert.-Butyl-5-brom-6-methyluracil
3-Isopropyl-5-brom-6-methyluracil
3-sec.-Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-ethan (Salze)
1-(4-Chlorphenoxy)-3,3-dimethyl-1-(1H-1,2,4-triazol-1-yl)-butan-2-on
N,N-Diallylchloracetamid
N-Isopropyl-2-chloracetanilid
N-(1-Methyl-propin-2-yl)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetatanilid

2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy)-N-methoxy-acetamid
2-(α-Naphthoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyl-trifluormethansulfonanilid
5-Acetamido-4-methyl-trifluormethansulfonanilid

2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid

2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxy-4'-nitro-phenylether
2-(3,4-Dichlorphenyl)-4-methyl-1,2,4-oxadiazolidin-3,5-dion
2-(3-tert.-Butylcarbamoyloxy-phenyl)-4-methyl-1,2,4-oxadiazolidin-3,5-dion
2-(3-i-Propylcarbamoyloxy-phenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfonat
2-Ethoxy-2,3-dihydro-3,3-dimethyl-5-benzofuranyl-(N-methyl-N-acetyl)-aminosulfonat

3,4-Dichlor-1,2-benzisothiazol
N-4-Chlorphenyl-allylbernsteinsäureimid
2-Methyl-4,6-dinitrophenol (Salze, Ester)
2-sec.-Butyl-4,6-dinitrophenol (Salze)
2-sec.-Butyl-4,6-dinitrophenol-acetat
2-tert.-Butyl-4,6-dinitrophenol-acetat

2-tert.-Butyl-4,6-dinitrophenol (Salze)
2-tert.-Butyl-5-methyl-4,6-dinitrophenol (Salze)
2-tert.-Butyl-5-methyl-4,6-dinitrophenol-acetat

2-sec.-Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff

1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4-(4'-Chlorphenoxy)-phenyl]-3,3-dimethyl-harnstoff
1-[4-(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-
-3,3-dimethyl-harnstoff

1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.-Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyloxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-methylsulfat
1,1'-Di-(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Alloyloxyamino)-propyliden]-5,5-dimethylcyclohexan-1,3-dion (Salze)

2-[1-(N-Allyloxyamino)-butyliden]-5,5-dimethylcyclohexan-1,3-dion (Salze)

2-[1-(N-Allyloxyamino)-butyliden]-5,5-dimethyl-4-methoxycarbonyl-cyclohexan-1,3-dion (Salze)

2-Chlorphenoxyessigsäure (Salze, Ester, Amide)

4-Chlorphenoxyessigsäure (Salze, Ester, Amide)

2,4-Dichlorphenoxyessigsäure (Salze, Ester, Amide)

2,4,5-Trichlorphenoxyessigsäure (Salze, Ester, Amide)

2-Methyl-4-chlorphenoxyessigsäure (Salze, Ester, Amide)

3,5,6-Trichlor-2-pyridinyl-oxyessigsäure (Salze, Ester, (Amide)

α-Naphthoxyessigsäuremethylester

2-(2-Methylphenoxy)-propionsäure (Salze, Ester, Amide)

2-(4-Chlorphenoxy)-propionsäure (Salze, Ester, Amide)

2-(2,4-Dichlorphenoxy)-propionsäure (Salze, Ester, Amide)

2-(2,4,5-Trichlorphenoxy)-propionsäure (Salze, Ester, Amide)

2-(2-Methyl-4-chlorphenoxy)-propionsäure (Salze, Ester, Amide)

4-(2,4-Dichlorphenoxy)-buttersäure (Salze, Ester, Amide)

4-(2-Methyl-4-chlorphenoxy)-buttersäure (Salze, Ester, Amide)

Cyclohexyl-3-(2,4-dichlorphenoxy)-acrylat

9-Hydroxyfluoren-carbonsäure-(9) (Salze, Ester)

2,3,6-Trichlorphenyl-essigsäure (Salze, Ester)

4-Chlor-2-oxo-benzothiazolin-3-yl-essigsäure (Salze, Ester)

Gibellerinsäure (Salze)

Dinatrium-methylarsonat

Mononatriumsalz der Methylarsonsäure

N-Phosphon-methyl-glycin (Salze)

N,N-Bis-(phosphonmethyl)-glycin (Salze)

2-Chlorethanphosphonsäure-2-chlorethylester

Ammonium-ethyl-carbamoyl-phosphonat

Di-n-butyl-1-n-butylamino-cyclohexyl-phosphonat
Trithiobutylphosphit
O,O-Diisopropyl-5-(2-benzosulfonylamino-ethyl)-phosphordithionat
2,3-Dihydro-5,6-dimethyl-1,4-dithiin-1,1,4,4-tetraoxid
5-tert.-Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.-Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäureethylester
2-[4-(5'-Jodpyridyl-2-oxy)-phenoxy]-propionsäure-n-butylester
2-Chlor-4-trifluormethylphenyl-3'-(2-fluorethoxy)-4'-nitro-phenylether

2-Chlor-4-trifluormethylphenyl-3(ethoxycarbonyl)methylthio-4-nitrophenylether

2,4,6-Trichlorphenyl-3(ethoxycarbonyl)methylthio-4-nitro-phenylether

2-[1-(N-ethoxamino)-butyliden]-5-(2-ethylthiopropyl)-3-hydroxy-cyclohexen-(2)-on-(1) (Salze)

2-[1-(N-ethoxamino)-butyliden]-5-(2-pheynlthiopropyl)-3-hydroxy-cyclohexen-(2)-on-(1) (Salze)

4-[4-(4'-Trifluormethyl)-phenoxy]-penten-2-carbonsäure-ethylester

2-Chlor-4-trifluormethyl-3'-methoxycarbonyl-4'-nitro-phenylether

2,4-Dichlorphenyl-3'-carboxy-4'-nitrophenylether (Salze)

4,5-Dimethoxy-2-(3-α,α,ß-trifluor-ß-bromethoxyphenyl)-3-(2H)-pyridazinon

2,4-Dichlorphenyl-3'-ethoxy-ethoxy-ethoxy-4'-nitrophenyl-ether

2,3-Dihydro-3,3-dimethyl-5-benzofuranyl-ethansulfonat

N-[4-Methoxy-6-methyl-1,3,5-triazin-2-yl-aminocarbonyl]-2-chlorbenzolsulfonamid

1(3-Chlor-4-ethoxyphenyl)-3,3-dimethylharnstoff

2-Methyl-4-chlorphenoxy-thioessigsäureethylester

2-Chlor-3,5-dijod-4-acetoxy-piridin

1-(4-[-2-(4-Methylphenyl)-äthoxy]-phenyl)-3-methyl-3-methoxy-harnstoff

2,6-Dimethyl-N-(pyrazol-1-yl-methylenoxymethyl)-2-chlor-acetanilid

2-Methyl-6-äthyl-N-(pyrazol-1-yl-methylenoxymethyl)-2-chlor-acetanilid

1-(α-2,4-Dichlorphenoxypropionsäure)-3-(O-methylcarbamoyl)-anilid

1-(α-2-Brom-4-chlorphenoxypropionsäure)-3-(O-methylcarbamoyl)-anilid
2-Methyl-6-äthyl-N-(pyrazol-1-yl-äthylenoxymethyl)-2-chloracetanilid
Methyl-N-dichlorfluormethylsulfenyl-(3-(N'-dichlorfluormethyl-sulfenyl-N'-phenylcarbamoyl-oxy)-phenyl)-carbamat
Methyl-N-dichlorfluormethylsulfenyl-(3-(N'-dichlorfluormethylsulfenyl-N'-3-methylphenylcarbamoyl-oxy)-phenyl)carbamat
N-(Pyrazol-1-yl-methyl)-pyrazol-1-yl-essigsäure-2,6-dimethylanilid
N-(Pyrazol-1-yl-methyl)-1,2,4-triazol-1-yl-essigsäure-2,6-dimethylanilid

2-(3-Trifluormethylphenyl)-4H-3,1-benzoxazin-4-on
2-(2-Thienyl)-4H-3,1-benzoxazin-4-on
2-(3-Pentafluorethoxyphenyl)-4H-3,1-benzoxazin-4-on
2-(3-Trifluormethylthio-penyl)-4H-3,1-benzoxazin-4-on
2-(3-Difluor-chlormethoxyphenyl)-4H-3,1-benzoxazin-4-on

5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-α,α,ß,ß)-tetrafluorethoxyphenyl)-4H-3,1-benzoxazin-4-on
5-Fluor-2-(3-α,α,ß,ß)-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-(phenyl)-4H-3,1-benzoxazin-4-on
5-Fluor-2-(3-difluormethoxyphenyl)-4H-3,1-benzoxazin-4-on
5-Chlor-2-(phenyl)-4H-3,1-benzoxazin-4-on
3-(3,5-Dichlorphenyl)-4-methoxycarbonyl-5-methylpyrazol
3-(3-Chlorphenyl)-4-methoxycarbonyl-5-methylpyrazol

3-(3-Fluorphenyl)-4-methoxycarbonyl-5-methylpyrazol
1-Acetyl-3-(3-fluorphenyl)-4-methoxycarbonyl-5-methylpyrazol
1-Acetyl-3-(3-chlorphenyl)-4-methoxycarbonyl-5-methylpyrazol
1-Acetyl-3-(3-bromphenyl)-4-methoxycarbonyl-5-methylpyrazol
1-Acetyl-3-(3,5-dichlorphenyl)-4-methoxycarbonyl-5-methylpyrazol
1-Acetyl-3-thienyl-4-methoxycarbonyl-5-methylpyrazol
N-3-Chlor-4-isopropylphenyl-thiolcarbaminsäuremethylester
N-3-Methyl-4-fluorphenyl-thiolcarbaminsäuremethylester
N-3-Chlor-4-isopentyl-phenyl-thiolcarbaminsäuremethylester
N-3-Chlor-4-difluormethoxyphenyl-thiolcarbaminsäuremethylester
N-3-Chlor-4-(1-chlorisopropyl)-phenyl-thiolcarbaminsäuremethylester
1-(2-Fluorphenyl)-3-methyl-5-iminoimidazolidin-2-on
1-(3-Isopropyl-phenyl)-3-methyl-5-iminoimidazolidin-2-on
1-(4-Isopropyl-phenyl)-3-methyl-5-iminoimidazolidin-2-on
1-[3-(1,1,2,2-Tetrafluor-ethoxy)-phenyl]-3-methyl-5-iminoimidazolidin-2-on
1-(3,4-Dichlorphenyl)-3-methyl-5-iminoimidazolidin-2-on
1-(3,4-Difluorphenyl)-3-methyl-5-iminoimidazolidin-2-on
6-Methyl-3-methoxy-5,6-dihydro-1,2,4,6-thiatriazin-5-on-1,1-dioxid
6-Methyl-3-methoxy-5,6-dihydro-1,2,4,6-thiatriazin-5-on-1,1-dioxid Na-Salz
6-n.Propyl-3-methoxy-5,6-dihydro-1,2,4,6-thiatriazin-5-on-1,1-dioxid
6-Methyl-3-ethoxy-5,6-dihydro-1,2,4,6-thiatriazin-5-on-1,1-dioxid
6-n.Propyl-3-ethoxy-5,6-dihydro-1,2,4,6-thiatriazin-5-on-1,1-dioxid Na-Salz

6-Methyl-3-isopropoxy-5,6-dihydro-1,2,4,6-thiatriazin-5-on-1,1-dioxid

6-n.Propyl-3-isopropoxy-5,6-dihydro-1,2,4,6-thiatriazin-5-on-1,1-dioxid

6-Iso-propyl-3-sek.butoxy-5,6-dihydro-1,2,4,6-thiatriazin-5-on-1,1-dioxid Na-Salz

N-3'-(2"-Chlor-4"-trifluormethyl-phenoxy)-6'-nitrobenzoyl-anthranilsäure

N-3'-(2"-Chlor-4"-trifluormethyl-phenoxy)-6'-nitrobenzoyl-anthranilsäure-methylester

N-3'-(2"-Chlor-4"-trifluormethyl-phenoxy)-6'-nitrobenzoyl-anthranilsäure-Na-Salz

N-3'-(2"-Chlor-4"-trifluormethyl-phenoxy)-6'-nitrobenzoyl-3-anthranilsäure

N-3'-(2"-Chlor-4"-trifluormethyl-phenoxy)-6'-nitrobenzoyl-3-methylanthranilsäure

N-3'-(2"-Chlor-4"-trifluormethyl-phenoxy)-6'-nitrobenzoyl-anthranilsäure

N-3'-(2",4"-Dichlorphenoxy)-6'-nitrobenzoylanthranilsäure

2-[3'-(2"-Chlor-4"-trifluormethyl-phenoxy)-6'-nitro-phenyl]4H-1,3-benzoxazin-4-on

2-[3'-(2"-Chlor-4"-trifluormethyl-phenoxy)-6'-nitro-phenyl]4H-1,3-8-methoxy-benzoxazin-4-on

Außerdem ist es nützlich, die neuen Verbindungen allein oder in Kombination mit anderen Herbiziden auch noch mit weiteren Pflanzenschutzmitteln gemischt gemeinsam auszubringen, beispeilsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

| Botanischer Name | Abkürzung in Tabelle | Deutscher Name | Englischer Name |
|---|---|---|---|
| Abutilon theophrasti | - | Chinesischer Hanf | velvet leaf |
| Amaranthus spp. | - | Fuchsschwanz-Arten | pigweed |
| Chenopodium album | - | Weißer Gänsefuß | common lambsquarters |
| Chrysanthemum spp. | Chrys. spp. | Saatwucherblumenarten | corn marigold |
| Desmdoium tortuosum | Desmod. tort. | | Florida beggarweed |
| Galeopsis tetrahit | - | Gemeiner Hohlzahn | hempnettle |
| Galium aparine | Galium apa. | Klettenlabkraut | catchweed bedstraw |
| Helianthum annuus | Heli. ann. | Sonnenblume | sunflowers |
| Ipomea spp. | - | Prunkwindearten | morningglory |
| Lamium spp. | - | Taubnesselarten | henbit |
| Malva neglecta | - | Weg-Malve | common mallow |
| Mercurialis annua | Mercu. annua | Einjähriges Bingelkraut | annual mercury |
| Oryza sativa | Oryza sat. | Reis | rice |
| Polygonum persicaria | - | Floh-Knöterich | ladysthumb, redshank |
| Sesbania exaltata | - | Turibaum | hemp sesbania |
| Sida spinosa | Sida spn. | | teaweed (prickly sida) |
| Sinapis alba | - | Weißer Senf | white mustard |
| Solanum nigrum | - | Schwarzer Nachtschatten | black nightshade |
| Triticum aestivum | Tritic. aest. | Weizen | wheat |
| Veronica persica | - | Persischer Ehrenpreis | - |

Patentansprüche

1. 5-Amino-1-phenyl-4-cyanpyrazol der Formel

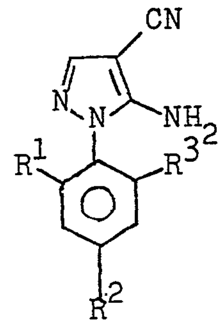

in der $R^1$, $R^2$, $R^3$ Chlor oder Brom bedeuten, mit der Maßgabe daß $R^1$, $R^2$ und $R^3$ nicht gleichzeitig Chlor bedeuten.

2. Herbizid enthaltend ein 5-Amino-1-phenyl-4-cyanpyrazol der Formel

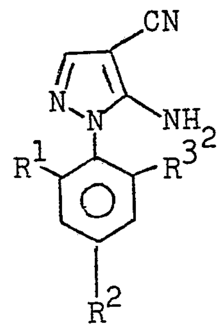

in der $R^1$, $R^2$, $R^3$ Chlor oder Brom bedeuten, mit der Maßgabe daß $R^1$, $R^2$ und $R^3$ nicht gleichzeitig Chlor bedeuten.

3. Herbizid enthaltend einen festen oder flüssigen Trägerstoff und ein 5-Amino-1-phenyl-4-cyanpyrazol der Formel

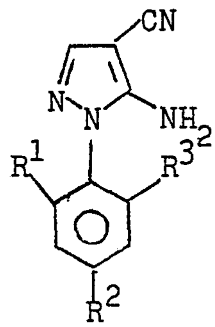

in der $R^1$, $R^2$, $R^3$ Chlor oder Brom bedeuten, mit der Maßgabe daß $R^1$, $R^2$ und $R^3$ nicht gleichzeitig Chlor bedeuten.

4. Verfahren zur Herstellung eines Herbizids, dadurch gekennzeichnet, daß man einen festen oder flüssigen Trägerstoff vermischt mit einem 5-Amino-1-phenyl-4--cyanpyrazol der Formel

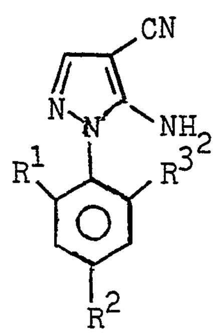

in der $R^1$, $R^2$, $R^3$ Chlor oder Brom bedeuten, mit der Maßgabe daß $R^1$, $R^2$ und $R^3$ nicht gleichzeitig Chlor bedeuten.

5. Verfahren zur Bekämpfung von unerwünschten Pflanzen, dadurch gekennzeichnet, daß man die Pflanzen oder den Boden behandelt mit einem 5-Amino-1-phenyl-4-cyanpyrazol der Formel

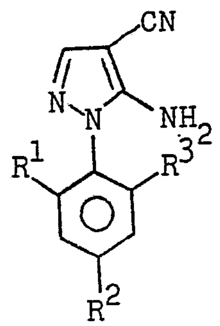

in der $R^1$, $R^2$, $R^3$ Chlor oder Brom bedeuten, mit der Maßgabe daß $R^1$, $R^2$ und $R^3$ nicht gleichzeitig Chlor bedeuten.

6. Verfahren zur Herstellung eines 5-Amino-1-phenyl-4--cyanpyrazols der Formel

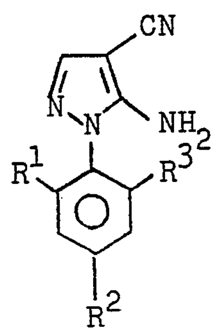

in der $R^1$, $R^2$, $R^3$ Chlor oder Brom bedeuten, mit der Maßgabe daß $R^1$, $R^2$ und $R^3$ nicht gleichzeitig Chlor bedeuten, dadurch gekennzeichnet, daß man ein 2,4,6-Trihalogenphenylhydrazin der Formel

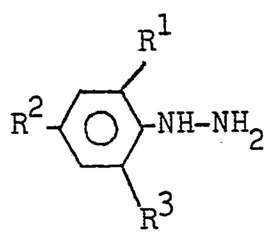

in der $R^1$, $R^2$, $R^3$ Chlor oder Brom bedeutet, mit einem Alkoximethylenmalodinitril der Formel

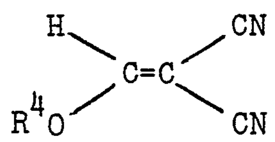

in der $R^4$ Alkyl mit bis zu 6 Kohlenstoffatomen bedeutet, in Gegenwart eines Lösungsmittels bei Temperaturen im Bereich von 50 bis 150°C umsetzt.

7. 5-Amino-1-phenyl-4-cyanpyrazol, ausgewählt aus der Gruppe bestehend aus 5-Amino-1-(2,4,6-tribromphenyl)-4-cyanpyrazol,
5-Amino-1-(2,4-dichlor-6-bromphenyl)-4-cyanpyrazol,
5-Amino-1-(2-chlor-4,6-dibromphenyl)-4-cyanpyrazol
und
5-Amino-1-(4-chlor-2,6-dibromphenyl)-4-cyanpyrazol.

8. Herbizid enthaltend ein 5-Amino-1-phenyl-4-cyanpyrazol, ausgewählt aus der Gruppe bestehend aus
5-Amino-1-(2,4,6-tribromphenyl)-4-cyanpyrazol,
5-Amino-1-(2,4-dichlor-6-bromphenyl)-4-cyanpyrazol,
5-Amino-1-(2-chlor-4,6-dibromphenyl)-4-cyanpyrazol
und
5-Amino-1-(4-chlor-2,6-dibromphenyl)-4-cyanpyrazol.

Patentansprüche für Österreich

1. Herbizid enthaltend ein 5-Amino-1-phenyl-4-cyan-pyrazol der Formel

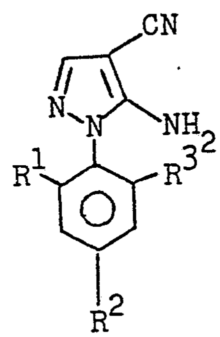

in der $R^1$, $R^2$, $R^3$ Chlor oder Brom bedeuten, mit der Maßgabe daß $R^1$, $R^2$ und $R^3$ nicht gleichzeitig Chlor bedeuten.

2. Herbizid enthaltend einen festen oder flüssigen Trägerstoff und ein 5-Amino-1-phenyl-4-cyanpyrazol der Formel

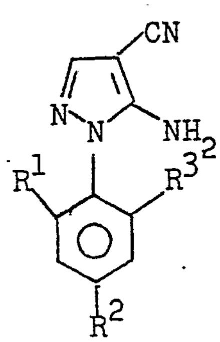

in der $R^1$, $R^2$, $R^3$ Chlor oder Brom bedeuten, mit der Maßgabe daß $R^1$, $R^2$ und $R^3$ nicht gleichzeitig Chlor bedeuten.

3. Verfahren zur Herstellung eines Herbizids, dadurch gekennzeichnet, daß man einen festen oder flüssigen Trägerstoff vermischt mit einem 5-Amino-1-phenyl-4-cyanpyrazol der Formel

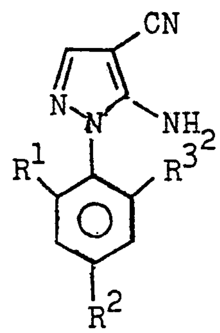

in der $R^1$, $R^2$, $R^3$ Chlor oder Brom bedeuten, mit der Maßgabe daß $R^1$, $R^2$ und $R^3$ nicht gleichzeitig Chlor bedeuten.

4. Verfahren zur Bekämpfung von unerwünschten Pflanzen, dadurch gekennzeichnet, daß man die Pflanzen oder den Boden behandelt mit einem 5-Amino-1-phenyl-4-cyan-pyrazol der Formel

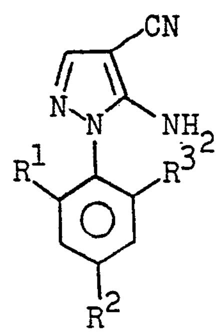

in der $R^1$, $R^2$, $R^3$ Chlor oder Brom bedeuten, mit der Maßgabe daß $R^1$, $R^2$ und $R^3$ nicht gleichzeitig Chlor bedeuten.

5. Verfahren zur Herstellung eines 5-Amino-1-phenyl-4--cyanpyrazols der Formel

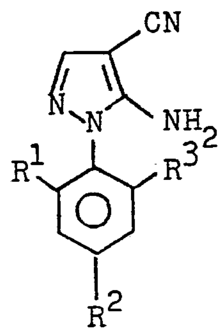

in der $R^1$, $R^2$, $R^3$ Chlor oder Brom bedeuten, mit der Maßgabe daß $R^1$, $R^2$ und $R^3$ nicht gleichzeitig Chlor bedeuten, dadurch gekennzeichnet, daß man ein 2,4,6-Trihalogenphenylhydrazin der Formel

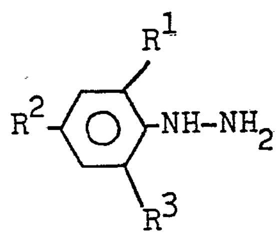

in der $R^1$, $R^2$, $R^3$ Chlor oder Brom bedeutet, mit einem Alkoximethylenmalodinitril der Formel

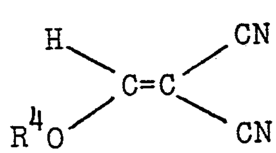

in der $R^4$ Alkyl mit bis zu 6 Kohlenstoffatomen bedeutet, in Gegenwart eines Lösungsmittels bei Temperaturen im Bereich von 50 bis 150°C umsetzt.

6. Herbizid enthaltend ein 5-Amino-1-phenyl-4-cyanpyrazol, ausgewählt aus der Gruppe bestehend aus 5-Amino-1-(2,4,6-tribromphenyl)-4-cyanpyrazol, 5-Amino-1-(2,4-dichlor-6-bromphenyl)-4-cyanpyrazol, 5-Amino-1-(2-chlor-4,6-dibromphenyl)-4-cyanpyrazol und 5-Amino-1-(4-chlor-2,6-dibromphenyl)-4-cyanpyrazol.

0053678

Europäisches Patentamt

# EUROPÄISCHER RECHERCHENBERICHT

Nummer der Anmeldung
EP 81 10 8477

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| E | EP - A - 0 034 945 (MAY AND BAKER) * Patentansprüche, Seite 54 - Zeilen 15-19 * ---- | 1-8 | C 07 D 231/38<br>A 01 N 43/56<br>C 07 C 121/82//<br>A 01 N 37/34 |
| | | | RECHERCHIERTE SACHGEBIETE (Int. Cl.3) |
| | | | C 07 D 231/00<br>A 01 N 43/00 |
| | | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| λ | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 18-03-1982 | CREMERS |

EPA form 1503.1 06.78